# EUROPÄISCHE PATENTSCHRIFT

(11) **EP 3 872 053 B1**
(45) Veröffentlichungstag und Bekanntmachung des Hinweises auf die Patenterteilung: **27.09.2023**
(21) Anmeldenummer: 20160130.9
(22) Anmeldetag: 28.02.2020
(51) Int. Cl.: C05F 17/40, C05F 17/979, C05F 17/986

(54) **EXTRAKTIONSVORRICHTUNG UND EXTRAKTIONSVERFAHREN FÜR EIN FERMENTATIONSMEDIUM**
EXTRACTION DEVICE AND EXTRACTION METHOD FOR FERMENTATION MEDIUM
DISPOSITIF D'EXTRACTION ET PROCÉDÉ D'EXTRACTION POUR UN MILIEU DE FERMENTATION

(43) Veröffentlichungstag der Anmeldung: 01.09.2021
(73) Patentinhaber: MICO-Systems GmbH, 85640 Putzbrunn (DE)
(72) Erfinder: LUNG, Gerhard, 70619 Stuttgart (DE); VON KÖNEMANN, Olaf, 85640 Putzbrunn (DE); BÖHM, Georg, 85640 Putzbrunn (DE)
(74) Vertreter: Kretschmann, Dennis

(56) Entgegenhaltungen:
- EP-A1- 1 208 068
- EP-B1- 1 414 938
- DE-A1-102007 023 654
- DE-A1-102015 220 315
- FR-A1- 2 904 308

## Beschreibung

### Technisches Gebiet

Die vorliegende Offenbarung bezieht sich auf eine Vorrichtung und ein Verfahren zum Extrahieren eines Fermentationsmediums, insbesondere eines ein Kompostmaterial enthaltenden Fermentationsmediums, aus einer Extraktionskammer, beispielsweise zur Bereitstellung einer mit Mikroorganismen befrachteten Flüssigkeit, welche sich in der Land- und Gartentechnik als Düngemittel oder Pflanzenschutzmittel verwenden lässt.

### Hintergrund

In der konventionellen Land- und Gartentechnik ist es üblich, die Wachstumsbedingungen von Pflanzen durch Ausbringung von Düngemitteln zu fördern und die Pflanzen durch Ausbringung von Herbiziden, Fungiziden und Pestiziden gegen Schädlinge zu schützen. Unter ökologischen Gesichtspunkten erweisen sich viele der zu diesem Zweck eingesetzten chemischen Substanzen als bedenklich. Insbesondere bei der großflächigen Anwendung derartiger Stoffe können nachteilige Auswirkungen für das betroffene Ökosystem, insbesondere eine Beeinträchtigung der Grundwasserqualität, auftreten. Auch kann die Qualität und der Geschmack der aus den so behandelten Pflanzen hergestellten Lebensmittel negativ beeinflusst werden.

Unter dem Eindruck dieser Probleme wurden bereits in der Vergangenheit auf pflanzlicher Basis hergestellte Düngemittel-Eluate entwickelt, die durch Auswaschen eines Fermentationsmediums, beispielsweise eines organischen Kompostmaterials, hergestellt werden und in flüssiger Form in Feld und Garten ausgebracht werden können. Ihre düngende und/oder pflanzenschützende Wirkung geht auf die in diesen Eluaten enthaltenen, aus dem Fermentationsmedium stammenden Mikroorganismen zurück.

Die Europäische Patentschrift EP 1 414 938 B1 beschreibt ein entsprechendes Verfahren und eine Vorrichtung zum Bereiten einer mit Mikroorganismen befrachteten Flüssigkeit, bei welchen ein Kompostmaterial in einer Anreicherungseinrichtung bereitgestellt wird, die über Leitungen und Pumpen mit einem Fermentationstank verbunden ist. Aus dem Fermentationstank wird über eine Zuführleitung Flüssigkeit von oben in die Anreicherungseinrichtung eingeführt. Die Flüssigkeit wäscht auf ihrem Weg hinunter durch die Anreicherungseinrichtung Mikroorganismen aus dem Kompostmaterial aus und wird unten aus der Anreicherungseinrichtung abgepumpt und über eine Abführleitung zurück in den Fermentationstank geführt. In dem Fermentationstank kann unter vorbestimmten Temperaturbedingungen eine weitere Konditionierung und Aufbereitung der mit den Mikroorganismen befrachteten Auswaschflüssigkeit ausgeführt werden, bis das Eluat schließlich als Düngemittel oder Pflanzenschutzmittel aus dem Fermentationstank entnommen werden kann.

Vorrichtungen zur Temperierung des Fermentationstanks und zur Sauerstoffzufuhr in den Fermentationstank sind auch in der Offenlegungsschrift DE 10 2007 023 654 A1 beschrieben.

Ein Fermentationstank mit Zu- und Ablauföffnungen in unterschiedlichen Höhenniveaus ist in der DE 10 2015 220 315 A1 beschrieben.

Die Offenlegungsschrift WO 01/05729 A1 offenbart einen Behandlungsprozess für organisches Abfallmaterial und eine entsprechende Vorrichtung für die anaerobische Zersetzung und aerobische Kompostierung. Eine Zersetzungsvorrichtung für organische Abfälle ist auch in der FR 2 904 308 A1 beschrieben.

### Überblick

Vor dem Hintergrund des Stands der Technik besteht Bedarf nach einer Vorrichtung und einem Verfahren, welche ein effizienteres Auswaschen des Fermentationsmediums und eine höhere Mikroorganismendichte in dem Eluat ermöglichen.

Diese Aufgabe wird mit einer Extraktionsvorrichtung für ein Fermentationsmedium gemäß Anspruch 1 bzw. mit einem Verfahren zum Extrahieren eines Fermentationsmediums gemäß Anspruch 12 gelöst. Die abhängigen Ansprüche beziehen sich auf vorteilhafte Weiterbildungen.

Eine Extraktionsvorrichtung für ein Fermentationsmedium umfasst eine Extraktionskammer, welche zum Aufnehmen eines Fermentationsmediums eingerichtet ist und einen unteren Abschnitt und einen oberen Abschnitt oberhalb des unteren Abschnitts umfasst, sowie eine Einspeiseeinrichtung, welche zum Einspeisen mindestens eines Gases und einer Flüssigkeit in den unteren Abschnitt der Extraktionskammer eingerichtet ist, sowie ferner eine Ablauföffnung, welche im oberen Abschnitt der Extraktionskammer angeordnet ist und dazu eingerichtet ist, eine mit Mikroorganismen befrachtete Auswaschflüssigkeit aus der Extraktionskammer abzuleiten.

Das mindestens eine Gas und die mindestens eine Flüssigkeit, welche von unten in die Extraktionskammer eingeleitet werden, durchsetzen das Fermentationsmedium auf dem Weg zur oberen Ablauföffnung. Auf diese Weise werden die Mikroorganismen besonders wirkungsvoll und vollständig aus dem Fermentationsmedium gelöst, sodass die aus der Extraktionskammer abgeleitete Auswaschflüssigkeit eine besonders hohe Mikroorganismendichte aufweist, die zu einer Erhöhung der düngenden und/oder pflanzenschützenden Wirkung des Eluats führt. Insbesondere kann das Einspeisen des Gases zusammen mit der Flüssigkeit von unten in die Extraktionskammer das effiziente Auswaschen des Fermentationsmediums zusätzlich fördern. Das Gas kann beispielsweise dazu beitragen, dass das Fermentationsmedium gelockert wird und die eingespeiste Flüssigkeit zersprudelt, sodass die eingespeiste Flüssigkeit in der Extraktionskammer über einen großen Oberflächenquerschnitt mit dem Fermentationsmedium in Kontakt tritt.

Als Fermentationsmedium kann im Rahmen der vorliegenden Offenbarung jedes organische Medium eingesetzt werden. Insbesondere kann das Fermentationsmedium ein Feststoff sein.

In einer Ausführungsform enthält das Fermentationsmedium zumindest anteilig ein Kompostmaterial. Das Kompostmaterial kann biologisch aktiv sein und eine Vielzahl lebender aerober Mikroorganismen umfassen. Als Kompostmaterial kann insbesondere Grüngutmaterial, welches optional auch mit Stallmistgaben angereichert sein kann, eingesetzt werden.

Gemäß einigen Ausführungsformen können dem Fermentationsmedium auch Zusätze beigefügt sein, welche eine Vermehrung der Mikroorganismen fördern. Als Zusätze können beispielsweise Melasse oder Vinasse oder Algenpräparate verwendet werden.

Als Extraktionskammer kann im Rahmen der vorliegenden Offenbarung jeder Behälter oder Raum Verwendung finden, der dazu eingerichtet ist, dass Fermentationsmedium aufzunehmen und auszuwaschen. Das Fermentationsmedium kann in der Extraktionskammer beispielsweise lose oder in flüssigkeits- und gasdurchlässigen Beuteln aufgenommen sein.

Im Rahmen der vorliegenden Offenbarung können die Begriffe "unten" und "oben" bzw. "unterer Abschnitt" und "oberer Abschnitt" eine relative Orientierung bezüglich einer Vertikalrichtung der Extraktionsvorrichtung bezeichnen. Insbesondere kann der untere Abschnitt im Betrieb der Extraktionsvorrichtung einem Untergrund, auf dem die Extraktionsvorrichtung direkt oder indirekt abgestützt ist, näher liegen als der obere Abschnitt. Die Vertikalrichtung kann der Richtung eines Schwerkraftfeldes entsprechen, sodass sich Massen unter Einwirkung der Schwerkraft entlang der Vertikalrichtung von oben nach unten, insbesondere aus dem oberen Abschnitt in den unteren Abschnitt, bewegen können.

Der obere Abschnitt kann dem unteren Abschnitt in der Extraktionskammer entgegengesetzt oder entgegengerichtet sein.

In einigen Ausführungsformen können weitere (mittlere) Abschnitte der Extraktionskammer zwischen dem unteren Abschnitt und dem oberen Abschnitt liegen und den oberen Abschnitt räumlich von dem unteren Abschnitt trennen.

Eine Einspeiseeinrichtung im Rahmen der Offenbarung kann jede Einrichtung sein, welche das Einspeisen des mindestens einen Gases und der mindestens einen Flüssigkeit in den unteren Abschnitt der Extraktionskammer ermöglicht, sodass das Gas und die Flüssigkeit mit dem in der Extraktionskammer aufgenommenen Fermentationsmedium in Kontakt kommen und das Fermentationsmedium auswaschen. Durch das Auswaschen kann sich die mit Mikroorganismen befrachtete Auswaschflüssigkeit bilden.

In einer Ausführungsform kann ein Gas und eine Flüssigkeit in die Extraktionskammer eingespeist werden. In anderen Ausführungsformen können mehrere Gase und/oder mehrere Flüssigkeiten in die Extraktionskammer eingespeist werden.

In einer Ausführungsform enthält das Gas Sauerstoff. Insbesondere kann das Gas Luft enthalten oder sein.

Das eingespeiste Gas, beispielsweise Luft bzw. Sauerstoff, trägt dazu bei, dass sich die Mikroorganismen aus dem Fermentationsmedium wirkungsvoll lösen und in hoher Dichte ausgewaschen werden können. Zudem fördert das Zuführen von Sauerstoff die Anreicherung aerober Mikroorganismen in der Auswaschflüssigkeit.

In einer Ausführungsform enthält die Flüssigkeit Wasser oder ist Wasser.

Durch eine Kombination von Sauerstoff und Wasser lässt sich das Fermentationsmedium besonders wirkungsvoll auswaschen und eine hohe Dichte aerober Mikroorganismen in dem Eluat erreichen.

In einer Ausführungsform kann die Einspeiseeinrichtung dazu eingerichtet sein, das Gas und/oder die Flüssigkeit mit einer vorbestimmten Flussrate in den unteren Abschnitt der Extraktionskammer einzuspeisen. Die Einspeiseeinrichtung kann dazu in einigen Ausführungsformen Ventile, insbesondere Drosselventile, und/oder Druckregler umfassen, um die vorbestimmte Flussrate des Gases und/oder der Flüssigkeit einzustellen.

Durch das Einstellen der Flussrate des Gases und/oder der Flüssigkeit lassen sich der Auswaschprozess und die Mikroorganismendichte gezielt und wirksam beeinflussen.

In einer Ausführungsform beträgt eine Flussrate des Gases mindestens 10 l/min, insbesondere mindestens 15 l/min oder mindestens 18 l/min.

Die Flussrate des Gases kann in einer Ausführungsform höchstens 30 l/min betragen, insbesondere höchstens 25 l/min oder höchstens 20 l/min.

In einer Ausführungsform beträgt eine Flussrate der Flüssigkeit mindestens 0,3 l/min, insbesondere mindestens 0,5 l/min oder mindestens 0,8 l/min.

Die Flussrate der Flüssigkeit kann in einer Ausführungsform höchstens 4 l/min betragen, insbesondere höchstens 3 l/min oder höchstens 2,5 l/min.

In einer Ausführungsform kann die Einspeiseeinrichtung dazu eingerichtet sein, das Gas und die Flüssigkeit separat in die Extraktionskammer einzuspeisen.

In einer anderen Ausführungsform ist die Einspeiseeinrichtung dazu eingerichtet, eine Mischung des Gases und der Flüssigkeit in die Extraktionskammer einzuspeisen.

Insbesondere kann die Einspeiseeinrichtung dazu eingerichtet sein, das Gas und die Flüssigkeit zu mischen, insbesondere in einem vorbestimmten Mischungsverhältnis zu mischen.

In einer Ausführungsform beträgt ein Mischungsverhältnis von Gas zu Flüssigkeit mindestens 5 zu 1, insbesondere mindestens 10 zu 1 oder mindestens 15 zu 1 oder mindestens 20 zu 1.

Das Mischungsverhältnis von Gas zu Flüssigkeit kann in einer Ausführungsform höchstens 35 zu 1 betragen, insbesondere höchstens 30 zu 1 betragen oder höchstens 25 zu 1 betragen.

Die Einspeiseeinrichtung kann dazu eingerichtet sein, das Gas und/oder die Flüssigkeit in einem Boden oder einem Bodenbereich der Extraktionskammer einzuspeisen.

In einer Ausführungsform umfasst die Einspeiseeinrichtung eine Membran, welche dazu eingerichtet ist, das Gas und/oder die Flüssigkeit in die Extraktionskammer einzuspeisen.

Mittels einer Membran lässt sich auf besonders vorteilhafte Weise ein Gemisch des Gases und der Flüssigkeit in die Extraktionskammer einspeisen.

Das Auswaschen des Fermentationsmediums lässt sich auch über eine Porenweite der Membran beeinflussen. Je kleiner die Porenweite, desto feinperliger ist das resultierende Gas-Flüssigkeits-Gemisch.

Als Porenweite kann im Rahmen der vorliegenden Offenbarung beispielsweise ein maximaler Durchmesser von Poren oder Öffnungen der Membran angesehen werden.

In einer Ausführungsform weist die Membran eine Porenweite von höchstens 1 mm auf, insbesondere von höchstens 0,8 mm oder höchstens 0,6 mm.

In einer Ausführungsform weist die Membran eine Porenweite von mindestens 0,1 mm auf, insbesondere mindestens 0,3 mm oder mindestens 0,5 mm.

Die Extraktionsvorrichtung umfasst in einer Ausführungsform ein erstes Siebelement und ein zweites Siebelement, welche die Extraktionskammer nach unten bzw. nach oben begrenzen, wobei die Einspeiseeinrichtung dazu eingerichtet ist, das Gas und/oder die Flüssigkeit durch das erste Siebelement hindurch in die Extraktionskammer einzuspeisen, und wobei das zweite Siebelement derart eingerichtet und angeordnet ist, dass die Auswaschflüssigkeit die Extraktionskammer durch das zweite Siebelement hindurch verlässt.

Insbesondere kann das zweite Siebelement der Ablauföffnung in Flussrichtung vorgeschaltet sein.

Die beiden Siebelemente bilden in einigen Ausführungsformen eine einfache und wirkungsvolle Begrenzung der Extraktionskammer innerhalb der Extraktionsvorrichtung. Insbesondere kann das erste Siebelement und/oder das zweite Siebelement reversibel entfernbar sein, um einen Zugang zum Inneren der Extraktionskammer zu ermöglichen und dadurch ein einfaches Beschicken der Extraktionskammer mit dem Fermentationsmedium zu gestatten.

In einer Ausführungsform weist das erste Siebelement und/oder das zweite Siebelement eine Maschengröße von mindestens 1 mm auf, insbesondere von mindestens 3 mm oder mindestens 5 mm.

Das erste Siebelement und/oder das zweite Siebelement kann in einer Ausführungsform eine Maschengröße von höchstens 10 mm aufweisen, insbesondere von höchstens 8 mm oder von höchstens 6 mm.

Als Maschengröße kann im Rahmen der vorliegenden Offenbarung beispielsweise ein maximaler Durchmesser von Maschen oder Öffnungen des ersten Siebelements bzw. des zweiten Siebelements angesehen werden.

In einer Ausführungsform kann das erste Siebelement oberhalb der Membran angeordnet sein, insbesondere der Membran in Flussrichtung nachgeschaltet sein.

Auf diese Weise kann das erste Siebelement die Membran vor einem Verstopfen mit dem Fermentationsmedium schützen. Gleichzeitig kann das von der Membran abgegebene Gas-Flüssigkeit-Gemisch durch die Maschen des ersten Siebelements hindurchtreten und das Fermentationsmedium auswaschen.

Als Ablauföffnung der Extraktionsvorrichtung kann im Rahmen der vorliegenden Offenbarung jede Öffnung im oberen Abschnitt der Extraktionskammer verstanden sein, die dazu ausgebildet und dazu geeignet ist, die über die Einspeiseeinrichtung zugeführte Flüssigkeit nach deren Kontakt mit dem Fermentationsmedium aus der Extraktionskammer abzuleiten.

Die Ablauföffnung kann als eine zusammenhängende Öffnung ausgebildet sein. In einer anderen Ausführungsform kann die Ablauföffnung mehrere Teilöffnungen, die möglicherweise räumlich getrennt sind, umfassen.

Gemäß einer Ausführungsform umfasst die Extraktionsvorrichtung ein Gehäuse, in welchem die Extraktionskammer aufgenommen ist.

Insbesondere kann zwischen einer Gehäuseinnenwand des Gehäuses und einer Außenwand der Extraktionskammer ein Fluidkanal, insbesondere ein zusammenhängender Fluidkanal, zur Aufnahme und Führung der aus der Extraktionskammer abgeleiteten Auswaschflüssigkeit ausgebildet sein.

Der Fluidkanal kann mit einer Ablaufvorrichtung ausgebildet sein, um die Auswaschflüssigkeit aus der Extraktionsvorrichtung abzuleiten, beispielsweise einem Fermentationstank zuzuleiten.

In einer Ausführungsform umfasst die Ablauföffnung einen Überlauf der Extraktionskammer.

Gemäß einer Ausführungsform umfasst die Extraktionsvorrichtung ein Filterelement, welches der Ablauföffnung in Flussrichtung vorgeschaltet ist und/oder unterhalb der Ablauföffnung angeordnet ist. Das Filterelement kann dazu eingerichtet sein, die Auswaschflüssigkeit vor dem Ableiten durch die Ablauföffnung zu filtern.

In einer Ausführungsform ist das Filterelement zwischen dem ersten Siebelement und dem zweiten Siebelement angeordnet. Insbesondere kann das Filterelement dem zweiten Siebelement in Flussrichtung vorgeschaltet sein.

In einer Ausführungsform umfasst das Filterelement einen metallischen Filter, insbesondere einen Filter aus einem Chrom-Nickel-Werkstoff.

Durch das Einstellen einer Maschengröße des Filterelements lässt sich der Auswaschprozess in einigen Ausführungsformen beeinflussen. Je kleiner die Maschengröße, desto weniger und desto kleinere Fermentationspartikel werden ausgeschwemmt.

Im Rahmen der vorliegenden Offenbarung kann die Maschengröße des Filterelements einen maximalen Durchmesser der Maschen bzw. Öffnungen des Filterelements bezeichnen.

Das Filterelement kann in einer Ausführungsform eine Maschengröße von mindestens 0,1 mm aufweisen, insbesondere von mindestens 0,3 mm oder mindestens 0,5 mm aufweisen.

Gemäß einer Ausführungsform kann das Filterelement eine Maschengröße von höchstens 1 mm aufweisen, insbesondere von höchstens 0,8 mm oder von höchstens 0,6 mm aufweisen. In einer Ausführungsform umfasst die Extraktionsvorrichtung zusätzlich eine Temperiereinrichtung, welche dazu eingerichtet ist, die Extraktionskammer und/oder das Gas und/oder die Flüssigkeit selektiv zu erwärmen und/oder zu kühlen.

Auf diese Weise lässt sich in der Extraktionskammer eine für die Mikroorganismen günstige Temperatur einstellen bzw. die Vermehrung der Mikroorganismen und damit die Mikroorganismendichte beeinflussen.

In einer Ausführungsform ist die Extraktionsvorrichtung zum lösbaren Aufsitzen auf einen Fermentationstank eingerichtet, wobei die Ablauföffnung der Extraktionsvorrichtung mit einer Einlassöffnung des Fermentationstanks fluidkoppelbar ist.

Auf diese Weise lässt sich die von der Extraktionsvorrichtung abgegebene Auswaschflüssigkeit auf effiziente und einfache Weise in den Fermentationstank überführen, in welchem die Mikroorganismen unter vorbestimmten Temperaturbedingungen vermehrt werden können.

In einer Weiterbildung kann die Ablauföffnung der Extraktionsvorrichtung mit der Einlassöffnung des Fermentationstanks direkt fluidkoppelbar sein, insbesondere ohne Rohr- und Schlauchleitungen fluidkoppelbar sein.

Das lösbare Aufsitzen der Extraktionsvorrichtung ermöglicht es in einigen Ausführungsformen zum einen, die Extraktionsvorrichtung zum vereinfachten Befüllen der Extraktionskammer von dem Fermentationstank zu lösen.

Zudem erfordert das Auswaschen des Fermentationsmediums aus der Extraktionsvorrichtung üblicherweise weniger Zeit als die sich daran anschließende Konditionierung der Auswaschflüssigkeit im Fermentationstank. Die Extraktionsvorrichtung kann in einigen Ausführungsform nach dem Auswaschen bereits von dem Fermentationstank entfernt und ggf. neu befüllt werden, während im Fermentationstank noch die weitere Konditionierung der Auswaschflüssigkeit erfolgt. Auf diese Weise lässt sich die Effizienz der Eluatherstellung insgesamt steigern.

Zudem können nacheinander mehrere Extraktionsvorrichtungen auf den Fermentationstank aufgesetzt werden, die gegebenenfalls auch mit unterschiedlichen Fermentationsmedien befüllt werden können, sodass sich in dem Fermentationstank die Auswaschflüssigkeit aus den mehreren Extraktionsvorrichtungen zusammenführen und/oder mischen lässt.

In einer Ausführungsform liegt die Ablauföffnung der Extraktionsvorrichtung oberhalb der Einlassung des Fermentationstanks.

Die Extraktionskammer und der Fermentationstank können in einigen Ausführungsformen derart eingerichtet und zueinander ausgerichtet sein, dass die Auswaschflüssigkeit allein unter dem Einfluss der Schwerkraft von der Ablauföffnung der Extraktionsvorrichtung in den Fermentationstank fließen kann.

Auf diese Weise lässt sich die Auswaschflüssigkeit auf besonders einfache und robuste Weise von der Extraktionsvorrichtung in den Fermentationstank überführen. Auf spezielle Rohrleitungen und Pumpen zur Überführung der Auswaschflüssigkeit in den Fermentationstank kann dadurch in einigen Ausführungsformen verzichtet werden, sodass der Gefahr einer Verstopfung von Rohrleitungen und Pumpen vorgebeugt wird und die Ausfallsicherheit der Extraktionsvorrichtung insgesamt gesteigert werden kann. Darin liegt ein unabhängiger Aspekt der vorliegenden Offenbarung.

Die Offenbarung bezieht sich daher auch auf ein System, welches eine Extraktionsvorrichtung mit einem oder allen der vorangehend beschriebenen Merkmale umfasst und zusätzlich einen Fermentationstank umfasst, wobei die Extraktionsvorrichtung zum lösbaren Aufsitzen auf den Fermentationstank eingerichtet ist und die Ablauföffnung der Extraktionsvorrichtung mit einer Einlassöffnung des Fermentationstanks fluidkoppelbar ist.

Gemäß einer Ausführungsform umfasst der Fermentationstank eine Temperiereinrichtung, welche dazu eingerichtet ist, die Auswaschflüssigkeit selektiv zu erwärmen und/oder zu kühlen.

Durch eine geeignete Temperierung der Auswaschflüssigkeit lassen sich die Bedingungen für die Vermehrung der Mikroorganismen in dem Fermentationstank gezielt einstellen und auf diese Weise die Mikroorganismendichte im Fermentationstank erhöhen.

Gemäß einer Ausführungsform umfasst der Fermentationstank eine Einspeiseeinrichtung zum Einspeisen eines Gases in den Fermentationstank.

In einer Ausführungsform umfasst das Gas Sauerstoff. Insbesondere kann das Gas Luft enthalten oder sein.

Durch eine gezielte Sauerstoffzufuhr in den Fermentationstank lässt sich die Mikroorganismendichte aerober Mikroorganismen gezielt erhöhen.

Ein Verfahren zum Extrahieren eines Fermentationsmediums umfasst ein Bereitstellen eines Fermentationsmediums in einer Extraktionskammer, ein Einspeisen mindestens eines Gases und einer Flüssigkeit in einen unteren Abschnitt der Extraktionskammer zum Ausbilden einer mit Mikroorganismen befrachteten Auswaschflüssigkeit aus dem Fermentationsmedium, und ein Ableiten der Auswaschflüssigkeit aus einem oberen Abschnitt der Extraktionskammer oberhalb des unteren Abschnitts.

Gemäß einer Ausführungsform enthält das Gas Sauerstoff. Insbesondere kann das Gas Luft enthalten oder sein.

Entsprechend einer Ausführungsform enthält die Flüssigkeit Wasser oder ist Wasser.

In einer Ausführungsform umfasst das Verfahren ein selektives Einstellen einer Flussrate des Gases und/oder der Flüssigkeit.

Gemäß einer Ausführungsform beträgt eine Flussrate des Gases mindestens 10 l/min, insbesondere mindestens 15 l/min oder mindestens 18 l/min.

In einer Ausführungsform kann eine Flussrate des Gases höchstens 30 l/min betragen, insbesondere höchstens 25 l/min betragen oder höchstens 20 l/min betragen

Gemäß einer Ausführungsform beträgt eine Flussrate der Flüssigkeit mindestens 0,3 l/min, insbesondere mindestens 0,5 l/min oder mindestens 0,8 l/min.

In einer Ausführungsform beträgt eine Flussrate der Flüssigkeit höchstens 4 l/min, insbesondere höchstens 3 l/min oder höchstens 2,5 l/min.

Gemäß einer Ausführungsform kann das Einspeisen ein Zuleiten einer Mischung des Gases und der Flüssigkeit in die Extraktionskammer umfassen.

Nach einer Ausführungsform umfasst das Verfahren ein Mischen des Gases und der Flüssigkeit vor dem Einspeisen in die Extraktionskammer, insbesondere in einem vorbestimmten Mischungsverhältnis.

Das Mischungsverhältnis von Gas zu Flüssigkeit kann in einer Ausführungsform mindestens 5 zu 1 betragen, insbesondere mindestens 10 zu 1 betragen oder mindestens 15 zu 1 betragen oder mindestens 20 zu 1 betragen.

Gemäß einer Ausführungsform beträgt das Mischungsverhältnis von Gas zu Flüssigkeit höchstens 35 zu 1, insbesondere höchstens 30 zu 1 oder höchstens 25 zu 1.

In einer Ausführungsform werden das Gas und/oder die Flüssigkeit über einen Boden oder Bodenbereich der Extraktionskammer in die Extraktionskammer eingespeist.

Gemäß einer Ausführungsform werden das Gas und/oder die Flüssigkeit durch eine Membran hindurch in die Extraktionskammer eingespeist.

Die Membran kann gemäß einer Ausführungsform eine Porenweite von höchstens 1 mm aufweisen, insbesondere höchstens 0,8 mm oder höchstens 0,6 mm.

In einer Ausführungsform kann die Membran eine Porenweite von mindestens 0,1 mm aufweisen, insbesondere mindestens 0,3 mm oder mindestens 0,5 mm

Das Einspeisen kann in einer Ausführungsform ein Zuleiten des Gases und/oder der Flüssigkeit durch ein erstes Siebelement hindurch in die Extraktionskammer umfassen.

Gemäß einer Ausführungsform kann das Ableiten der Auswaschflüssigkeit aus der Extraktionskammer durch ein zweites Siebelement hindurch erfolgen.

Das erste Siebelement und/oder das zweite Siebelement können in einer Ausführungsform eine Maschengröße von mindestens 1 mm aufweisen, insbesondere von mindestens 3 mm oder von mindestens 5 mm aufweisen.

Gemäß einer Ausführungsform weist das erste Siebelement und/oder das zweite Siebelement eine Maschengröße von höchstens 10 mm auf, insbesondere von höchstens 8 mm oder von höchstens 6 mm.

In einer Ausführungsform umfasst das Verfahren ein Filtern der Auswaschflüssigkeit vor dem Ableiten der Auswaschflüssigkeit aus der Extraktionskammer, insbesondere mittels eines Filterelements, durch das hindurch die Auswaschflüssigkeit aus der Extraktionskammer abgeleitet wird.

Das Filterelement kann einen metallischen Filter umfassen, insbesondere aus einem Chrom-Nickel-Werkstoff.

In einer Ausführungsform weist das Filterelement eine Maschengröße von mindestens 0,1 mm auf, insbesondere von mindestens 0,3 mm oder mindestens 0,5 mm.

Das Filterelement kann in einer Ausführungsform eine Maschengröße von höchstens 1 mm aufweisen, insbesondere von höchstens 0,8 mm oder von höchstens 0,6 mm

Gemäß einer Ausführungsform umfasst das Verfahren ein Temperieren der Extraktionskammer und/oder des Gases und/oder der Flüssigkeit, insbesondere ein selektives Erwärmen und/oder ein selektives Kühlen der Extraktionskammer und/oder des Gases und/oder der Flüssigkeit.

Gemäß einer Ausführungsform wird die Auswaschflüssigkeit in einen Fermentationstank abgeleitet, insbesondere in einen unterhalb der Extraktionskammer angeordneten Fermentationstank.

Als Fermentationstank kann Rahmen der vorliegenden Offenbarung jeder Behälter verstanden werden, welche dazu eingerichtet ist, die von der Extraktionsvorrichtung abgeleitete Auswaschflüssigkeit aufzunehmen und gegebenenfalls weiter zu konditionieren.

Gemäß einer Ausführungsform kann die Auswaschflüssigkeit allein unter Einwirkung der Schwerkraft in den Fermentationstank abgeleitet werden.

In einer Ausführungsform umfasst das Verfahren ein lösbares Aufsetzen der Extraktionskammer auf den Fermentationstank.

Gemäß einer Ausführungsform umfasst das Verfahren ein Temperieren der Auswaschflüssigkeit und/oder des Fermentationstanks, insbesondere ein selektives Erwärmen und/oder ein selektives Kühlen der Auswaschflüssigkeit und/oder des Fermentationstanks.

Gemäß einer Ausführungsform umfasst das Verfahren ein Einspeisen eines Gases in den Fermentationstank.

In einer Ausführungsform enthält das Gas Sauerstoff. Insbesondere kann das Gas Luft enthalten oder sein.

### Kurze Beschreibung der Zeichnungen

Die Merkmale und zahlreichen Vorteile der Extraktionsvorrichtung bzw. des Extraktionsverfahrens gemäß der vorliegenden Offenbarung lassen sich am besten anhand einer detaillierten Beschreibung von Ausführungsformen unter Bezugnahme auf die anliegenden Zeichnungen verstehen, in denen:
- Fig. 1: eine schematische Darstellung einer Extraktionsvorrichtung gemäß einer Ausführungsform zeigt;
- Fig. 2: eine schematische Querschnittsdarstellung einer Extraktionsvorrichtung gemäß einer Ausführungsform zeigt;
- Fig. 3: eine schematische Querschnittsdarstellung einer Extraktionsvorrichtung im Zusammenwirken mit einem Fermentationstank gemäß einer Ausführungsform zeigt; und
- Fig. 4: ein Flussdiagramm eines Extraktionsverfahrens gemäß einer Ausführungsform darstellt.

### Beschreibung von Ausführungsformen

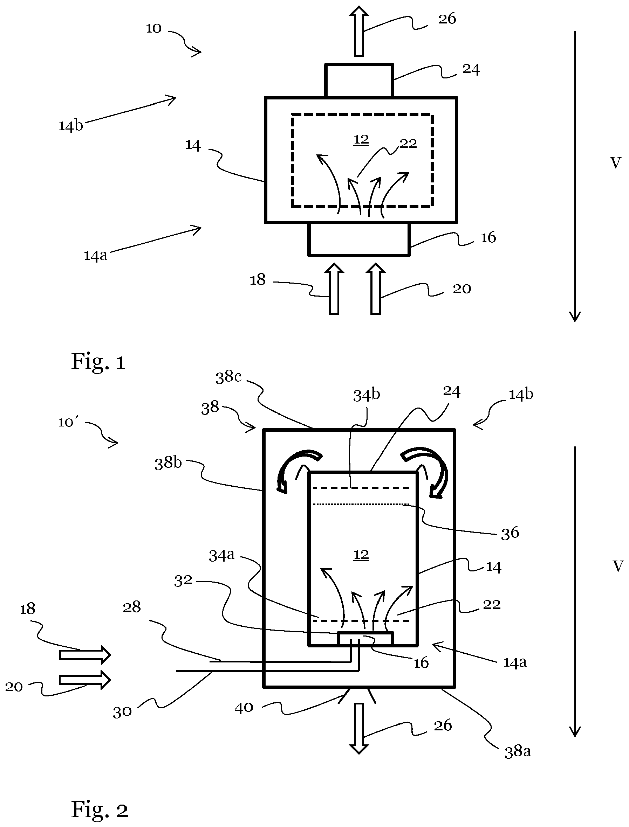

Figur 1 ist eine schematische Darstellung einer Extraktionsvorrichtung 10, wie sie beispielsweise zum Auswaschen eines Fermentationsmediums 12 verwendet werden kann, um eine mit Mikroorganismen befrachtete Auswaschflüssigkeit bereitzustellen, die - gegebenenfalls nach weiterer Konditionierung und Aufbereitung - beispielsweise als organisches Düngemittel oder organisches Pflanzenschutzmittel Verwendung finden kann.

Das Fermentationsmedium kann insbesondere zumindest anteilig aus einem Kompostmaterial gebildet sein. Als Kompostmaterial hat sich beispielsweise Grüngutmaterial, das optional auch mit Stallmistgaben angereichert sein kann, als geeignet erwiesen. Besonders hohe Extraktionsergebnisse können auch mit Rindenkompostmaterial erreicht werden, das sich zudem durch eine hohe Regenerationsfähigkeit auszeichnet.

Die Auswaschflüssigkeit kann nützliche aerobe Mikroorganismen aus der Gruppe der Antagonisten umfassen, die beispielsweise gegen parasitäre Pilzkrankheiten wirksam sind. Sie eignet sich u.a. zur Behandlung von Rasentragschichten sowie zur Wachstumsförderung von Nutzpflanzen im Bereich der Landwirtschaft, insbesondere im Bereich des Gemüsebaus. Weitere Anwendungsmöglichkeiten betreffen den Wein- und Hopfenanbau, die Teichpflege, Desinfektion, aber auch die Reduzierung der Geruchsemissionen und des Volumens von Mülldeponien. Es handelt sich dabei jedoch nur um veranschaulichende Beispiele. Die Extraktionsvorrichtung 10 kann in einer Vielzahl von Extraktionsprozessen vorteilhaft eingesetzt werden.

Zu den weiteren Einzelheiten möglicher Fermentationsmedien einschließlich gegebenenfalls eingesetzter Zusatzstoffe wird auf die europäische Anmeldeschrift EP 1 414 938 B1 sowie auf die Offenlegungsschrift DE 10 2007 023 654 A1 verwiesen.

Die in Figur 1 gezeigte Extraktionsvorrichtung 10 umfasst eine Extraktionskammer 14, die dazu eingerichtet ist, das Fermentationsmedium 12, beispielsweise das Kompostmaterial, lose oder verpackt in einem gas- und flüssigkeitsdurchlässigen Beutel oder Behälter in ihrem Inneren aufzunehmen.

Wie in Figur 1 weiter gezeigt ist, umfasst die Extraktionsvorrichtung 10 auch eine Einspeiseeinrichtung 16, welche in einem unteren Abschnitt 14a der Extraktionskammer 14 angeordnet ist und dazu eingerichtet ist, mindestens einen Gasstrom 18, beispielsweise Sauerstoff oder Luft, und mindestens einen Flüssigkeitsstrom 20, beispielsweise Wasser, in den unteren Abschnitt 14a der Extraktionskammer 14 einzuspeisen. Die Einspeiseeinrichtung 16 kann darüber hinaus in einigen Ausführungsformen Drosselventile (in Fig. 1 nicht gezeigt) umfassen, welche es ermöglichen, einen Druck und/oder eine Flussrate des Gasstroms 18 und einen Druck und oder eine Flussrate des Flüssigkeitsstroms 20 selektiv und unabhängig voneinander einzustellen.

Die Einspeiseeinrichtung 16 kann den Gasstrom 18 und den Flüssigkeitsstrom 20 in einigen Ausführungsformen als zwei voneinander räumlich getrennte Ströme in die Extraktionskammer 14 einspeisen. In anderen Ausführungsformen ist die Einspeiseeinrichtung 16 dazu eingerichtet, den Gasstrom 18 und den Flüssigkeitsstrom 20 zu mischen, beispielsweise in einem vorbestimmten Mischungsverhältnis zu mischen, und das resultierende Gas-Flüssigkeits-Gemisch 22 in die Extraktionskammer 14 einzuspeisen.

Beispielsweise kann die Einspeiseeinrichtung 16 dazu eingerichtet sein, die Flussrate des Gasstroms 18 in einem Bereich zwischen 18 l/min und 20 l/min einzustellen und die Flussrate des Flüssigkeitsstroms 20 in einem Bereich zwischen 0,85 l/min und 2,4 l/min einzustellen.

Das Gas-Flüssigkeits-Gemisch 22 steigt in der Extraktionskammer 14 von dem unteren Abschnitt 14a aus aufwärts in Richtung auf einen oberen Abschnitt 14b der Extraktionskammer 14, welcher dem unteren Abschnitt 14a entgegengesetzt ist, und durchsetzt auf seinem Weg das Fermentationsmedium 12, so dass das Gas-Flüssigkeits-Gemisch 22 das Fermentationsmedium 12 unter Bildung einer mit Mikroorganismen befrachteten Auswaschflüssigkeit auswäscht.

Da das Gas-Flüssigkeits-Gemisch 22 von unten aus in die Extraktionskammer 14 eintritt und das darin aufgenommene Fermentationsmedium 12 durchsetzt und durchmischt, erfolgt eine besonders wirksame und effiziente Auswaschung. Der Gasanteil in dem Gas-Flüssigkeits-Gemisch 22 führt zu einem Auflockern und einer Oberflächenvergrößerung des Fermentationsmediums 12, wodurch die Flüssigkeit an dem Fermentationsmedium 12 besonders wirksam und entlang einer vergrößerten Oberfläche angreifen kann, sodass die Wirksamkeit der Auswaschung zusätzlich gesteigert wird.

In dem oberen Abschnitt 14b umfasst die Extraktionskammer 14 eine Ablauföffnung 24, welche dazu eingerichtet ist, die mit Mikroorganismen befrachtete Auswaschflüssigkeit aus der Extraktionskammer 14 abzuleiten, zum Beispiel als Auswaschflüssigkeitsstrom 26 einem Fermentationstank (in Figur 1 nicht gezeigt) zuzuleiten.

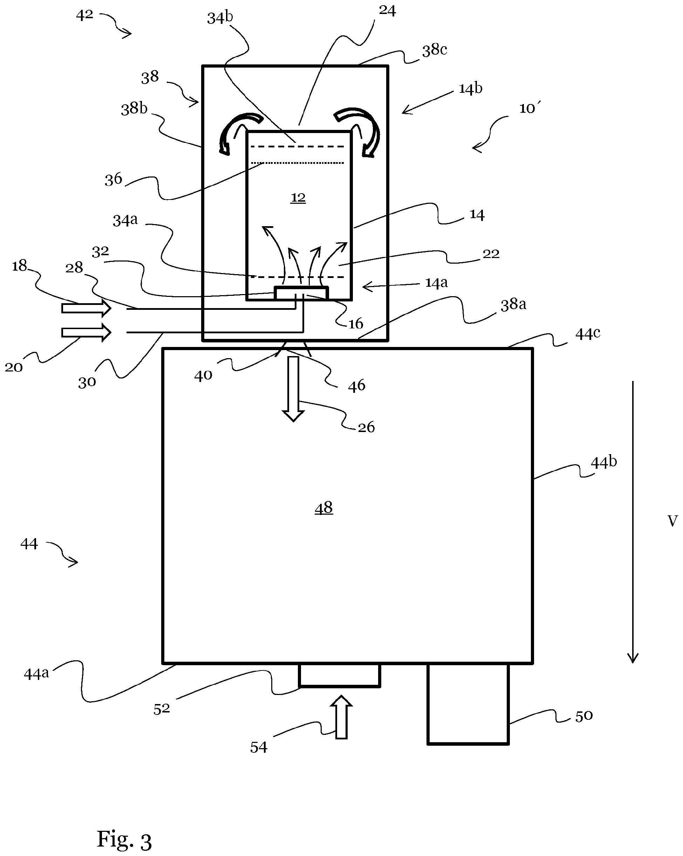

In Figur 1 (und ebenso in den nachfolgend unter Bezugnahme auf die Figuren 2 und 3 beschriebenen Ausführungsformen) zeigen die relativen Begriffe "unten" und "oben" eine Orientierung bezüglich einer Vertikalrichtung V der Extraktionsvorrichtung 10 im Betrieb an, wobei der untere Abschnitt 14a einem Untergrund, auf dem die Extraktionsvorrichtung 10 im Betrieb direkt oder indirekt gelagert ist, näher ist als der obere Abschnitt 14b. Die Vertikalrichtung V entspricht im Betrieb der Extraktionsvorrichtung 10 auch der Richtung einer Schwerebeschleunigung, die auf die Extraktionsvorrichtung 10 wirkt.

Das Gas-Flüssigkeits-Gemisch 22 steigt also von dem unteren Abschnitt 14a aus entgegen der die Schwerkraft anzeigenden Vertikalrichtung V durch die Extraktionskammer 14 und das darin aufgenommene Fermentationsmedium 12 aufwärts, wäscht dabei das Fermentationsmedium 12 wirkungsvoll aus und verlässt die Extraktionskammer 14 als Auswaschflüssigkeitsstrom 26 über die in dem oberen Abschnitt 14b der Extraktionskammer 14 ausgebildete Ablauföffnung 24.

Figur 2 zeigt eine Ausführungsform einer Extraktionsvorrichtung 10' in einer schematischen Querschnittsansicht. Die Extraktionsvorrichtung 10' gleicht der vorangehend unter Bezugnahme auf Figur 1 beschriebenen Extraktionsvorrichtung 10 weitgehend, und einander entsprechende Komponenten sind mit denselben Bezugszeichen versehen. Im folgenden wird daher die Extraktionsvorrichtung 10' nur insoweit detailliert beschrieben, wie sie sich von der Extraktionsvorrichtung 10 unterscheidet.

Die Extraktionsvorrichtung 10' umfasst ein hohlzylindrisches Gehäuse 38 mit einer kreisförmigen Bodenwand 38a an ihrer Unterseite, Seitenwänden 38b entlang ihrer Peripherie und einer kreisförmigen Deckelwand 38c an ihrer Oberseite. Im Innern des Gehäuses 38 ist die Extraktionskammer 14 als nach oben offener Hohlzylinder aufgenommen und lässt sich daher nach Entfernung der Deckelwand 38c des Gehäuses 38 von oben durch die Ablauföffnung 24 schnell und einfach mit dem Fermentationsmedium 14 beschicken. Zwischen der Bodenwand 38a des Gehäuses 38 und der Unterseite der Extraktionskammer 14 sowie zwischen den Seitenwänden 38b des Gehäuses 38 und den Seitenwänden der Extraktionskammer 14 ist ein zusammenhängender Hohlraum gebildet, der als Fließkanal für die aus der Ablauföffnung 24 austretende Auswaschflüssigkeit dienen kann.

Der Einspeiseeinrichtung 16 in dem unteren Abschnitt 14a der Extraktionskammer 14 werden über eine fluiddicht durch die Seitenwand 38b des Gehäuses 38 geführte Gaszuführleitung 28, die beispielsweise mit einem Luftkompressor oder einer Sauerstoffquelle (nicht gezeigt) verbunden ist, der Gasstrom 18 und über eine ebenfalls fluiddicht durch die Seitenwand 38b des Gehäuses 38 geführte Flüssigkeitszuführleitung 30, die beispielsweise mit einem Frischwasseranschluss (nicht gezeigt) verbunden ist, der Flüssigkeitsstrom 20 zugeführt. Sowohl die Gaszuführleitung 28 als auch die Flüssigkeitszuführleitung 30 können mit Drosselventilen oder Absperrventilen (nicht gezeigt), beispielsweise Magnetventilen, ausgebildet sein, welche ein Einstellen des Gasstroms 18 bzw. des Flüssigkeitsstrom 20 auf vorbestimmte Flussraten ermöglichen.

Die Einspeiseeinrichtung 16 ist im Bodenbereich der Extraktionskammer 14 angeordnet und mischt den Gasstrom 18 und den Flüssigkeitsstrom 20 in einem vorbestimmten Mischungsverhältnis, beispielsweise im Verhältnis 15:1 oder 20:1, und gibt das resultierende Gas-Flüssigkeits-Gemisch 22 über eine mit Poren versehene Membran 32, beispielsweise eine Kunststoffmembran, in den Innenraum der Extraktionskammer 14 ab. Die Poren der Membran 32 können beispielsweise einen Durchmesser von 0,5 mm oder weniger aufweisen.

Wie in der Querschnittdarstellung der Figur 2 zusätzlich zu erkennen ist, weist die Extraktionsvorrichtung 10' in der gezeigten Ausführungsform in der Extraktionskammer 14 ein erstes (unteres) Siebelement 34a und ein zweites (oberes) Siebelement 34b auf, die einen Aufnahmeraum für das Fermentationsmedium 12 nach unten und oben begrenzen. Die Siebelemente 34a und 34b können beispielsweise Maschen mit einem Durchmesser von 3 mm aufweisen. Das erste Siebelement 34a verhindert, dass das Fermentationsmedium 12 unmittelbar auf der Membran 32 der Einspeiseeinrichtung 16 aufliegt und diese verstopft. Das zweite Siebelement 34b verhindert im Betrieb ein Ausschwemmen des Fermentationsmediums 12 durch die Ablauföffnung 24. Das zweite Siebelement 34b kann insbesondere lösbar ausgebildet sein und aus der Extraktionskammer 14 entnommen werden, um ein Befüllen der Extraktionskammer 14 mit dem Fermentationsmedium 12 von oben durch die Ablauföffnung 24 hindurch zu vereinfachen.

Die Querschnittdarstellung der Figur 2 zeigt auch ein Filterelement 36, welches im oberen Abschnitt 14b der Extraktionskammer 14 unmittelbar unterhalb des zweiten Siebelements 34b angeordnet ist dazu eingerichtet ist, Schwebeteilchen aus der Auswaschflüssigkeit zu filtern. Das Filterelement 36 kann beispielsweise aus einem Chrom-Nickel-Werkstoff mit einer Stärke von 0,8 mm und einer Maschenweite von 0,5 mm x 0,5 mm ausgebildet sein.

Das aus der Membran 32 austretende Gas-Flüssigkeits-Gemisch 22 durchströmt das erste Siebelement 34a und wäscht das in der Extraktionskammer 14 aufgenommene Fermentationsmedium 12 unter Bildung einer mit Mikroorganismen befrachteten Auswaschflüssigkeit aus, wie vorangehend unter Bezugnahme auf Figur 1 beschrieben wurde. Die Auswaschflüssigkeit wird durch das Filterelement 36 gefiltert und gelangt durch die Maschen des zweiten Siebelements 34b zur Ablauföffnung 24, die ist der Ausführungsform der Figur 2 als Überlauf ausgebildet ist, wie schematisch durch die beiden Pfeile angedeutet ist, die ein Überlaufen der Auswaschflüssigkeit über den Rand der Extraktionskammer 14 andeuten.

Die aus der Extraktionskammer 14 austretende Auswaschflüssigkeit fließt in dem zwischen der Außenwand der Extraktionskammer 14 und der Innenwand des Gehäuses 38 gebildeten Fluidkanal lang der Vertikalrichtung V abwärts und sammelt sich oberhalb der Bodenwand 38a der Extraktionsvorrichtung 10', von wo aus sie die Extraktionsvorrichtung 10' durch ein verschließbares Auslassventil 40 als Auswaschflüssigkeitsstrom 26 verlassen kann. Der von der Extraktionsvorrichtung 10' abgegebene Auswaschflüssigkeitsstrom 26 kann beispielsweise einem Fermentationstank zugeführt werden, wie nachfolgend unter Bezugnahme auf Figur 3 in weiteren Einzelheiten beschrieben ist.

Figur 3 zeigt in einer schematischen Querschnittdarstellung ein Extraktionssystem 42 gemäß einer Ausführungsform, welches die Extraktionsvorrichtung 10', wie sie vorangehend unter Bezugnahme auf die Figuren 1 und 2 beschrieben wurde, und einen Fermentationstank 44 umfasst.

Der Fermentationstank 44 ist in der gezeigten Ausführungsform als zylinderförmiger und geschlossener Hohlbehälter zur Aufnahme eines Auswaschflüssigkeitsvolumens 48 ausgebildet und umfasst an seiner Unterseite eine kreisförmige Bodenwand 44a, entlang seiner Peripherie Seitenwände 44b und an seiner Oberseite eine kreisförmige Deckelwand 44c. Sein Volumen kann beispielsweise 1000 1 betragen.

Die Extraktionsvorrichtung 10' sitzt in der Darstellung der Figur 3 mit ihrer Bodenwand 38a lösbar auf einer Oberseite des Fermentationstanks 44 auf, beispielsweise auf seiner Deckelwand 44c, sodass die Extraktionskammer 14 und insbesondere deren Ablauföffnung 24 in Vertikalrichtung V oberhalb des Fermentationstanks 44 liegen. Die Extraktionsvorrichtung 10' ist lösbar mit dem Fermentationstank 44 koppelbar, derart dass das Auslassventil 40 in der Bodenwand 38a der Extraktionsvorrichtung 10' mit einer Einlassöffnung 46 in der Deckelwand 44c des Fermentationstanks 44 in Fluidverbindung steht.

Im Betrieb kann daher die aus der Extraktionskammer 14 austretende Auswaschflüssigkeit bei geöffnetem Auslassventil 40 der Extraktionsvorrichtung 10' und geöffneter Einlassöffnung 46 des Fermentationstanks 44 allein unter dem Einfluss der Schwerkraft als Auswaschflüssigkeitsstrom 26 von der Extraktionskammer 14 in den Fermentationstank 44 einfließen, ohne dass dafür Pumpen oder Kompressoren nötig sind. Da die Extraktionsvorrichtung 10' mit ihrer Bodenwand 38a unmittelbar auf der Deckelwand 44c des Fermentationstanks 44 aufsitzt, kann auch auf externe Verbindungsleitungen oder Verbindungsschläuche zwischen der Extraktionsvorrichtung 10' und dem Fermentationstank 44 weitgehend verzichtet werden, sodass das Extraktionssystem 43 insgesamt besonders robust und wartungsunempfindlich ist.

In typischen Anwendungen des Extraktionssystems 42 erfolgt das Auswaschen über eine kürzere Zeitspanne, beispielsweise 7 Stunden bis 20 Stunden, als die Verweildauer der Auswaschflüssigkeit im Fermentationstank, die bis zu 60 Stunden betragen kann.

Beispielsweise lässt sich ein Fermentationstank 44 mit einem Fassungsvermögen von 1000 1 bei einer Flussrate des Flüssigkeitsstroms 20 von 0,85 l/min in ca. 20 Stunden befüllen, bei einer Flussrate des Flüssigkeitsstroms 20 von 2,4 l/min in ca. 7 Stunden. Der Gasstrom 18 kann dabei jeweils eine Flussrate im Bereich von 18-20 l/min aufweisen.

Das Extraktionssystem 42 bietet den Vorteil, dass die Extraktionsvorrichtung 10' nach dem erfolgten Auswaschen von dem Fermentationstank 44 getrennt, gesäubert und neu befüllt werden kann, während die Auswaschflüssigkeit 48 im Fermentationstank 44 zur Herstellung eines Eluats weiter konditioniert wird, beispielsweise geeignet temperiert oder mit Zusätzen versehen wird. Gegebenenfalls kann in der Zwischenzeit auch eine weitere Extraktionsvorrichtung, gegebenenfalls mit einem anderen Fermentationsmedium, auf den Fermentationstank 44 aufgesetzt und ausgewaschen werden. Die Effizienz der Eluatherstellung lässt sich auf diese Weise insgesamt steigern.

Die im Fermentationstank 44 aufgenommene Auswaschflüssigkeit 48 kann in einigen Ausführungsformen mittels einer Temperiereinrichtung 50 temperiert, beispielsweise selektiv erwärmt oder gekühlt werden, um die Auswaschflüssigkeit 48 auf eine vorbestimmte Temperatur, welche für die Vermehrung der Mikroorganismen geeignet ist, einzustellen. Beispielsweise kann die Temperiereinrichtung 50 derart angesteuert werden, dass die Temperatur der Auswaschflüssigkeit im Bereich von konstant 20-25 °C gehalten wird.

Über eine Gaseinspeiseeinrichtung 52 des Fermentationstanks 44, welche beispielsweise in der Bodenwand 44a des Fermentationstank 44 angeordnet sein kann, kann der Auswaschflüssigkeit 48 im Fermentationstank 44 gezielt ein Gasstrom 54, beispielsweise Luft von einem Kompressor (nicht gezeigt) oder Sauerstoff von einem Gastank (nicht gezeigt), zugeführt werden, um die Vermehrung der Mikroorganismen im Fermentationstank 44 und damit die Herstellung eines für die Düngung oder den Pflanzenschutz wirksamen Eluats zusätzlich zu fördern.

Zu den weiteren Einzelheiten und Möglichkeiten der Ausgestaltung der Extraktionsvorrichtung und des Fermentationstanks verweisen wir auf die Europäische Patentschrift EP 1 414 938 B1 sowie die Offenlegungsschrift DE 10 2007023 654 A1.

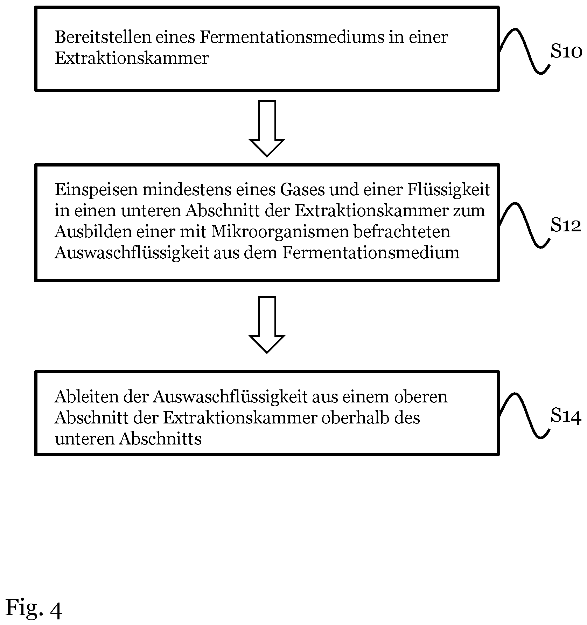

Figur 4 zeigt ein Flussdiagramm eines Verfahrens zum Extrahieren eines Fermentationsmediums gemäß einer Ausführungsform.

In einem ersten Schritt S10 umfasst das Verfahren ein Bereitstellen eines Fermentationsmediums in einer Extraktionskammer, beispielsweise in einer Extraktionskammer, wie sie vorangehend unter Bezugnahme auf die Ausführungsformen der Figuren 1 bis 3 beschrieben wurde.

In einem nachfolgenden zweiten Schritt S12 umfasst das Verfahren ein Einspeisen mindestens eines Gases und mindestens einer Flüssigkeit in einen unteren Abschnitt der Extraktionskammer zum Ausbilden einer mit Mikroorganismen befrachteten Auswaschflüssigkeit aus dem Fermentationsmedium.

In einem nachfolgenden dritten Schritt S14 umfasst das Verfahren ein Ableiten der Auswaschflüssigkeit aus einem oberen Abschnitt der Extraktionskammer oberhalb des unteren Abschnitts.

Die Beschreibung dieser Ausführungsformen und Figuren dienen lediglich der Erläuterung der Extraktionsvorrichtung bzw. des Extraktionsverfahrens der vorliegenden Offenbarung, soll sie aber nicht beschränken. Der Schutzumfang ergibt sich aus den anliegenden Ansprüchen.

## Patentansprüche

1. Extraktionsvorrichtung (10, 10') für ein Fermentationsmedium (12) mit:
einer Extraktionskammer (14), welche zum Aufnehmen eines Fermentationsmediums (12) eingerichtet ist und einen unteren Abschnitt (14a) und einen oberen Abschnitt (14b) oberhalb des unteren Abschnitts (14a) umfasst;
einer Einspeiseeinrichtung (16), welche zum Einspeisen mindestens eines Gases (18) und einer Flüssigkeit (20) in den unteren Abschnitt (14a) der Extraktionskammer (14) eingerichtet ist;
einer Ablauföffnung (24), welche im oberen Abschnitt (14b) der Extraktionskammer (14) angeordnet ist und dazu eingerichtet ist, eine mit Mikroorganismen befrachtete Auswaschflüssigkeit (26, 48) aus der Extraktionskammer (14) abzuleiten.

2. Extraktionsvorrichtung (10, 10') nach Anspruch 1, wobei das Gas (18) Sauerstoff enthält, insbesondere Luft enthält oder ist, und/oder die Flüssigkeit (20) Wasser enthält oder ist.

3. Extraktionsvorrichtung (10, 10') nach einem der vorangehenden Ansprüche, bei welcher die Einspeiseeinrichtung (16) dazu eingerichtet ist, das Gas (18) und/oder die Flüssigkeit (20) mit einer vorbestimmten Flussrate in den unteren Abschnitt (14a) der Extraktionskammer (14) einzuspeisen.

4. Extraktionsvorrichtung (10, 10') nach einem der vorangehenden Ansprüche, bei welcher die Einspeiseeinrichtung (16) dazu eingerichtet ist, das Gas (18) und die Flüssigkeit (20) zu mischen, insbesondere in einem vorbestimmten Mischungsverhältnis zu mischen.

5. Extraktionsvorrichtung (10, 10') nach einem der vorangehenden Ansprüche, bei welcher die Einspeiseeinrichtung (16) eine Membran (32) umfasst, welche dazu eingerichtet ist, das Gas (18) und/oder die Flüssigkeit (20) in die Extraktionskammer (14) einzuspeisen.

6. Extraktionsvorrichtung (10, 10') nach einem der vorangehenden Ansprüche mit einem ersten Siebelement (34a) und einem zweiten Siebelement (34b), welche die Extraktionskammer (14) nach unten bzw. nach oben begrenzen, wobei die Einspeiseeinrichtung (16) dazu eingerichtet ist, das Gas (18) und/oder die Flüssigkeit (20) durch das erste Siebelement (34a) in die Extraktionskammer (14) einzuspeisen, und wobei das zweite Siebelement (34b) derart eingerichtet und angeordnet ist, dass die Auswaschflüssigkeit (26, 48) die Extraktionskammer (14) durch das zweite Siebelement (34b) verlässt.

7. Extraktionsvorrichtung (10, 10') nach Anspruch 5 und 6, bei welcher das erste Siebelement (34a) oberhalb der Membran (32) angeordnet ist.

8. Extraktionsvorrichtung (10, 10') nach einem der vorangehenden Ansprüche mit einem Filterelement (36), welches der Ablauföffnung (24) in Flussrichtung vorgeschaltet ist und dazu eingerichtet ist, die Auswaschflüssigkeit (26, 48) vor dem Ableiten durch die Ablauföffnung (24) zu filtern.

9. Extraktionsvorrichtung (10, 10') nach Anspruch 8 in Verbindung mit Anspruch 7 oder 6, bei welcher das Filterelement (36) zwischen dem ersten Siebelement (34a) und dem zweiten Siebelement (34b) angeordnet ist.

10. Extraktionsvorrichtung (10, 10') nach einem der vorangehenden Ansprüche, welche zum lösbaren Aufsitzen auf einen Fermentationstank (44) eingerichtet ist, wobei die Ablauföffnung (24) der Extraktionsvorrichtung (10, 10') mit einer Einlassöffnung (46) des Fermentationstanks (44) fluidkoppelbar ist.

11. System (42) mit:
einer Extraktionsvorrichtung (10, 10') nach einem der vorangehenden Ansprüche; und
einem Fermentationstank (44);
wobei die Extraktionsvorrichtung (10, 10') zum lösbaren Aufsitzen auf den Fermentationstank (44) eingerichtet ist und die Ablauföffnung (24) der Extraktionsvorrichtung mit einer Einlassöffnung (46) des Fermentationstanks (44) fluidkoppelbar ist.

12. Verfahren zum Extrahieren eines Fermentationsmediums (12), umfassend:
Bereitstellen eines Fermentationsmediums (12) in einer Extraktionskammer (14);
Einspeisen mindestens eines Gases (18) und einer Flüssigkeit (20) in einen unteren Abschnitt (14a) der Extraktionskammer (14) zum Ausbilden einer mit Mikroorganismen befrachteten Auswaschflüssigkeit (26, 48) aus dem Fermentationsmedium (12); und
Ableiten der Auswaschflüssigkeit (26, 48) aus einem oberen Abschnitt (14b) der Extraktionskammer (14) oberhalb des unteren Abschnitts (14a).

13. Verfahren nach Anspruch 12, umfassend ein selektives Einstellen einer Flussrate des Gases (18) und/oder der Flüssigkeit (20).

14. Verfahren nach Anspruch 12 oder 13, bei welchem die Auswaschflüssigkeit (26, 48) in einen Fermentationstank (44) abgeleitet wird, insbesondere in einen unterhalb der Extraktionskammer (14) angeordneten Fermentationstank (44).

15. Verfahren nach Anspruch 14, umfassend ein lösbares Aufsetzen der Extraktionskammer (14) auf den Fermentationstank (44).

## Claims

1. An extraction apparatus (10, 10') for a fermentation medium (12) comprising:
an extraction chamber (14) configured to receive a fermentation medium (12) and comprising a lower portion (14a) and an upper portion (14b) above the lower portion (14a);
a feed device (16) configured to feed at least one gas (18) and one liquid (20) into the lower portion (14a) of the extraction chamber (14);
a discharge opening (24) arranged in the upper portion (14b) of the extraction chamber (14) and configured to drain a washout liquid (26, 48), loaded with microorganisms, from the extraction chamber (14).

2. The extraction apparatus (10, 10') according to claim 1, wherein the gas (18) contains oxygen, in particular contains air or is air, and/or the liquid (20) contains water or is water.

3. The extraction apparatus (10, 10') according to one of the preceding claims, wherein the feed device (16) is configured to feed the gas (18) and/or the liquid (20) at a predetermined flow rate into the lower portion (14a) of the extraction chamber (14).

4. The extraction apparatus (10, 10') according to one of the preceding claims, wherein the feed device (16) is configured to mix the gas (18) and the liquid (20), in particular to mix them in a predetermined mixing ratio.

5. The extraction apparatus (10, 10') according to one of the preceding claims, in which the feed device (16) comprises a membrane (32) configured to feed the gas (18) and/or the liquid (20) into the extraction chamber (14).

6. The extraction apparatus (10, 10') according to one of the preceding claims, having a first screen element (34a) and a second screen element (34b) which bound the extraction chamber (14) downwardly and upwardly, respectively, wherein the feed device (16) is configured to feed the gas (18) and/or the liquid (20) through the first screen element (34a) into the extraction chamber (14), and wherein the second screen element (34b) is configured and arranged such that the washout liquid (26, 48) leaves the extraction chamber (14) through the second screen element (34b).

7. The extraction apparatus (10, 10') according to claim 5 and 6, in which the first screen element (34a) is arranged above the membrane (32).

8. The extraction apparatus (10, 10') according to any one of the preceding claims, having a filter element (36) which is positioned upstream of the discharge opening (24) in the direction of flow and is configured to filter the washout liquid (26, 48) before it is drained through the discharge opening (24).

9. The extraction apparatus (10, 10') according to claim 8 in conjunction with claim 7 or 6, wherein the filter element (36) is arranged between the first screen element (34a) and the second screen element (34b).

10. The extraction apparatus (10, 10') according to one of the preceding claims, which is configured to be detachably seated on a fermentation tank (44), wherein the discharge opening (24) of the extraction apparatus (10, 10') is fluidically couplable to an inlet opening (46) of the fermentation tank (44).

11. A system (42) having:
an extraction apparatus (10, 10') according to one of the preceding claims; and
a fermentation tank (44);
wherein the extraction apparatus (10, 10') is configured to be detachably seated on the fermentation tank (44) and the discharge opening (24) of the extraction apparatus is fluidically couplable to an inlet opening (46) of the fermentation tank (44).

12. A method for extracting a fermentation medium (12), comprising:
providing a fermentation medium (12) in an extraction chamber (14);
feeding at least one gas (18) and one liquid (20) into a lower portion (14a) of the extraction chamber (14) to form a washout liquid (26, 48), loaded with microorganisms, from the fermentation medium (12); and
draining the washout liquid (26, 48) from an upper portion (14b) of the extraction chamber (14) above the lower portion (14a).

13. The method according to claim 12, comprising selectively adjusting a flow rate of the gas (18) and/or the liquid (20).

14. The method according to claim 12 or 13, in which the washout liquid (26, 48) is drained into a fermentation tank (44), in particular into a fermentation tank (44) arranged below the extraction chamber (14).

15. The method according to claim 14, comprising detachably fitting the extraction chamber (14) on the fermentation tank (44).

## Revendications

1. Dispositif d'extraction (10, 10') pour un milieu de fermentation (12) avec :
une chambre d'extraction (14) qui est conçue pour le logement d'un milieu de fermentation (12) et qui comprend une portion inférieure (14a) et une portion supérieure (14b) au-dessus de la portion inférieure (14a) ;
un dispositif d'alimentation (16) qui est conçu pour l'introduction d'au moins un gaz (18) et un liquide (20) dans la portion inférieure (14a) de la chambre d'extraction (14) ;
une ouverture d'évacuation (24), qui est disposée dans la portion supérieure (14b) de la chambre d'extraction (14) et qui est conçue pour évacuer le liquide de lavage (26, 48) chargé en micro-organismes hors de la chambre d'extraction (14).

2. Dispositif d'extraction (10, 10') selon la revendication 1, dans lequel le gaz (18) contient de l'oxygène, plus particulièrement contient ou est de l'air et/ou le liquide (20) contient ou est de l'eau.

3. Dispositif d'extraction (10, 10') selon l'une des revendications précédentes, dans lequel le dispositif d'alimentation (16) est conçu pour introduire le gaz (18) et/ou le liquide (20) avec un débit déterminé dans la portion inférieure (14a) de la chambre d'extraction (14).

4. Dispositif d'extraction (10, 10') selon l'une des revendications précédentes, dans lequel le dispositif d'alimentation (16) est conçu pour mélanger le gaz (18) et le liquide (20), plus particulièrement avec un rapport de mélange prédéterminé.

5. Dispositif d'extraction (10, 10') selon l'une des revendications précédentes, dans lequel le dispositif d'alimentation (16) comprend une membrane (32) qui est conçue pour introduire le gaz (18) et/ou le liquide (20) dans la chambre d'extraction (14).

6. Dispositif d'extraction (10, 10') selon l'une des revendications précédentes, avec un premier élément de tamisage (34a) et un deuxième élément de tamisage (34b) qui délimitent la chambre d'extraction (14) vers le bas ou vers le haut, dans lequel le dispositif d'alimentation (16) est conçu pour introduire le gaz (18) et/ou le liquide (20) à travers le premier élément de tamisage (34a) dans la chambre d'extraction (14) et dans lequel le deuxième élément de tamisage (34b) est conçu et disposé de façon à ce que le liquide de lavage (26, 48) quitte la chambre d'extraction (14) à travers le deuxième élément de tamisage (34b).

7. Dispositif d'extraction (10, 10') selon la revendication 5 et 6, dans lequel le premier élément de tamisage (34a) est disposé au-dessus de la membrane (32).

8. Dispositif d'extraction (10, 10') selon l'une des revendications précédentes, avec un élément de filtrage (36) qui est disposé en amont de l'ouverture d'évacuation (24) dans la direction du flux et qui est conçu pour filtrer le liquide de lavage (26, 48) avec son évacuation à travers l'ouverture d'évacuation (24).

9. Dispositif d'extraction (10, 10') selon la revendication 8, en lien avec la revendication 7 ou 6, dans lequel l'élément de filtrage (36) est disposé entre le premier élément de tamisage (34a) et le deuxième de tamisage (34b).

10. Dispositif d'extraction (10, 10') selon l'une des revendications précédentes, qui est conçu pour être posé de manière amovible sur un réservoir de fermentation (44), dans lequel l'ouverture d'évacuation (24) du dispositif d'extraction (10, 10') peut être couplée de manière fluidique avec une ouverture d'entrée (46) du réservoir de fermentation (44).

11. Système (42) avec :
un dispositif d'extraction (10, 10') selon l'une des revendications précédentes ; et
un réservoir de fermentation (44) ;
dans lequel le dispositif d'extraction (10, 10') est conçu pour être posé de manière amovible sur le réservoir de fermentation (44) et l'ouverture d'évacuation (24) du dispositif d'extraction (10, 10') peut être couplée de manière fluidique avec une ouverture d'entrée (46) du réservoir de fermentation (44).

12. Procédé d'extraction d'un milieu de fermentation (12) comprenant :
la mise à disposition d'un milieu de fermentation (12) dans une chambre d'extraction (14) ;
l'introduction d'au moins un gaz (18) et d'un liquide (20) dans une portion inférieure (14a) de la chambre d'extraction (14) afin de former un liquide de lavage (26, 48) chargé en micro-organismes à partir du milieu de fermentation (12) ; et
dérivation du liquide de lavage (26, 48) hors d'une portion supérieure (14b) de la chambre d'extraction (14) au-dessus de la portion inférieure (14a).

13. Procédé selon la revendication 12, comprenant un réglage sélectif d'un débit du gaz (18) et/ou du liquide (20).

14. Procédé selon la revendication 12 ou 13, dans lequel le liquide de lavage (26, 48) est dérivé vers un réservoir de fermentation (44), plus particulièrement vers un réservoir de fermentation (44) disposé en dessous de la chambre d'extraction (14).

15. Procédé selon la revendication 14, comprenant une pose amovible de la chambre d'extraction (14) sur le réservoir de fermentation (44).
